# FASCICULE DE BREVET EUROPEEN

(11) **EP 1 675 605 B1**
(45) Date de publication et mention de la délivrance du brevet: **09.04.2008**
(21) Numéro de dépôt: 04786353.5
(22) Date de dépôt: 26.08.2004
(51) Int. Cl.: A61K 36/899, A61K 36/75, A61K 36/11, A61P 37/04

(54) **COMPOSITION PHARMACEUTIQUE UTILISABLE NOTAMMENT EN TANT QU'ANTIVIRAL, ANTIBACTERIEN ET POUR STIMULER LES DEFENSES IMMUNITAIRES**
PHARMAZEUTISCHE ZUSAMMENSETZUNG BEISPIELSWEISE ZUR VERWENDUNG ALS ANTIVIRALES ODER ANTIBAKTERIELLES MITTEL UND ZUR STIMULIERUNG VON IMMUNABWEHR
PHARMACEUTICAL COMPOSITION WHICH CAN BE USED, FOR EXAMPLE, AS AN ANTIVIRAL OR ANTIBACTERIAL AGENT AND TO STIMULATE IMMUNE DEFENCES

(30) Priorité: 26.08.2003 FR 0310162
(43) Date de publication de la demande: 05.07.2006
(73) Titulaire: Eto, Bruno, 62000 Arras (FR)
(72) Inventeur: ETO, Bruno, F-62000 Arras (FR); PYEBI-OYOUBI, Pierre, Gabon (GA); LOUMA-EYOUGHA, Alphonse, Gabon (GA)
(74) Mandataire: Keib, Gérard
(86) Numéro de dépôt international: PCT/FR2004/002191
(87) Numéro de publication internationale: WO 2005/021019

(56) Documents cités:
- EP-A- 0 503 208
- US-A- 5 382 430
- US-A- 5 989 556
- DATABASE BIOSIS [Online] BIOSCIENCES INFORMATION SERVICE, PHILADELPHIA, PA, US; 1994, KAO SHING-FEN ET AL: "Immunostimulation by Alsophila spinulosa extract fraction VII of both humoral and cellular immune responses" XP002275791 Database accession no. PREV199598189960 & ANTICANCER RESEARCH, vol. 14, no. 6B, 1994, pages 2439-2443, ISSN: 0250-7005
- DATABASE WPI Section Ch, Week 200358 Derwent Publications Ltd., London, GB; Class D21, AN 2003-611648 XP002275792 & JP 2003 192531 A (NOEVIR KK) 9 juillet 2003 (2003-07-09)

## Description

La présente invention concerne une composition pharmaceutique renfermant en tant que principe actif, le mélange d'au moins un extrait d'au moins une graminée, d'un extrait d'au moins une rutacée, et d'un extrait d'au moins une ptéridophyte. Cette composition est destinée notamment à stimuler les défenses immunitaires et est utilisable comme antiviral et comme antibactérien.

Le système immunitaire comprend un ensemble de cellules spécialisées soumises à de multiples mécanismes de contrôle assurant leur renouvellement, leur activation, et leur différentiation, indispensables à un niveau normal d'immunocompétence. Le rôle du système immunitaire est de reconnaître le soi et le non soi pour éliminer les agents pathogènes et les tumeurs spontanées. Toute déplétion cellulaire, toute dérégulation immunitaire ou tout déficit fonctionnel est susceptible de favoriser la survenue de manifestations pathologiques caractérisées par des agressions microbiennes et virales.

Le tissu sanguin constitue un des éléments actifs du système de défense immunitaire. Parmi les types de cellules participants à la défense immunitaire on compte les lymphocytes T, lymphocytes B, les cellules K, les macrophages et les anticorps. Le tissu sanguin sain grâce à son système immunitaire peut lutter contre les agressions extérieures grâce aux moyens mis à sa disposition. Néanmoins, certaines cellules impliquées dans le système immunitaire comme les lymphocytes T peuvent eux-mêmes subir des agressions voir des destructions par des virus comme le HIV.

Ces agressions se traduisent par un effet suppresseur sur les défenses immunitaires entraînant une baisse des résistances aux agents pathogènes et notamment une augmentation de l'incidence de certaines maladies dites maladies opportunistes au SIDA.

Les maladies opportunistes au SIDA sont d'origines diverses. Les infections bactériennes et fongiques sont les plus courantes. Les traitements par les antifongiques et les antibactériens sont les plus utilisés pour lutter contre ces maladies opportunistes.

Les antibiotiques sont pour la plupart des substances naturelles issues de champignons microscopiques ou des bactéries du sol. L'arrivée de la pénicilline, puis de la streptomycine vers les années 40 a ouvert l'ère des antibiotiques. Leur utilisation a permis de sauver des millions de vie. L'apparition de la résistance aux antibiotiques est aujourd'hui reconnue comme un problème majeur de santé publique. La multirésistance est due au fait que ces agents pathogènes sont soumis régulièrement à l'action de plusieurs familles d'antibiotiques.

Dans la lutte contre les résistances, il est devenu urgent de développer des nouvelles approches allant de la biologie moléculaire à la recherche stratégique, qui visent à explorer systématiquement les ressources de la nature, plus particulièrement des antibiotiques issues des plantes.

L'un des buts de l'invention est donc de proposer une nouvelle composition pharmaceutique qui agit non seulement comme un immunorégulateur stimulant plus particulièrement, non seulement le système immunitaire, mais peut aussi être utilisée comme un antibactérien et comme un antiviral.

Ce but est atteint selon l'invention par une composition pharmaceutique renfermant au moins un extrait d'au moins une graminée plus un extrait d'au moins une rutacée, plus un extrait d'au moins une ptéridophyte, lesdits extraits étant des extraits de paspalum conjugatum, de fagara heitzii et de cyathea aethiopica.

Cette composition pharmaceutique est utilisable pour stimuler les défenses immunitaires, en particulier celles affaiblies par la maladie du VIH-SIDA.

Cette composition pharmaceutique est également utilisable pour lutter contre les infections bactériennes et virales, ou utilisable en tant qu'antifongique.

La famille des graminées compte plusieurs espèces. Les plantes de la famille des graminées sont surtout utilisées pour les affections cardiaques, les contusions, les entorses et les luxations. Parmi les genres de graminées utilisables selon l'invention on peut citer à titre d'exemple le genre *paspalum.* Comme matériel végétal utilisable on peut citer celui provenant de l'espèce *conjugatum.* Comme matériel végétal on peut utiliser les feuilles comme la plante entière soit sous forme des extraits des plantes fraîches ou sous forme des extraits des plantes séchés.

La famille des rutacées compte également plusieurs espèces. Les plantes de la famille des rutacées sont surtout utilisées traditionnellement pour les propriétés antirhumatismales analgésiques, contre les palpitations cardiaques et parfois pour narcotiser les poissons dans les rivières.

Parmi les genres de rutacées utilisables selon l'invention on peut citer à titre d'exemple le genre *fagara*. Comme matériel végétal utilisable on peut citer celui provenant des espèces végétales *fagara heitzii.* Comme matériel végétal on peut utiliser les extraits des feuilles, des écorces et des racines.

La famille des ptéridophytes compte également plusieurs espèces. Les plantes de la famille des ptéridophytes sont surtout utilisées traditionnellement pour les propriétés ulcéreuses gastriques. Parmi les genres de ptéridophytes utilisables selon l'invention on peut citer à titre d'exemple le genre *cyathea*. Comme matériel végétal utilisable on peut citer celui provenant des espèces végétales *cyathea aethiopica, cyathea manniana.* Comme matériel végétal on peut utiliser les extraits des feuilles, des tiges et des racines.

Toute méthode d'extraction connue de l'homme du métier peut être utilisée pour préparer l'extrait *paspalum,* de *fagara,* et de cyathea utilisable selon l'invention. On peut, en particulier, citer les extraits alcooliques, notamment éthanoliques ou encore hydroalcooliques. Ainsi, dans une première étape on broie le matériel végétal dans une solution aqueuse à froid, dans une deuxième étape les particules en suspension sont éliminées de la solution aqueuse issue de la première étape. Dans la troisième étape on stérilise la solution aqueuse issue de première étape. Dans la troisième étape on stérilise la solution aqueuse issue de la deuxième étape. Cette solution aqueuse correspond à l'extrait. D'autre part, la première étape peut avantageusement être remplacée par une opération de congélation des fragments des végétaux (par exemple à -20°C), suivie d'une extraction aqueuse à froid reprenant la deuxième et la troisième étape ci-dessus décrites. Le produit de cette extraction aqueuse peut également être lyophilisé pour obtenir un extrait sec (en moyenne selon la nature de la source initiale, on retrouve entre 3 et 80 g de matière sèche par litre d'extrait aqueux).

Un exemple de préparation d'extrait de graminée, plus de rutacée, plus de ptéridophytes utilisables selon l'invention est donné par ailleurs dans les exemples. La quantité d'extrait utilisable selon l'invention est fonction de l'effet recherché et peu donc varier dans une large mesure. Pour donner un ordre de grandeur, on peut utiliser un extrait sec ou liquide ou lyophilisé de graminée plus particulièrement *paspalum conjugatum,* tel que défini précédemment en une quantité représentant de 0,0001% à 30 % du poids total de la composition et préférentiellement en une quantité représentant 0,001 % à 15 % du poids total de la composition. On peut utiliser un extrait sec ou liquide ou lyophilisé de rutacée plus particulièrement la *fagara heitzii,* tel que défini précédemment en une quantité représentant de 0,0001% à 99 % du poids total de la composition et préférentiellement en une quantité représentant 0,001 % à 77 % du poids total de la composition. On peut utiliser un extrait sec ou liquide ou lyophilisé de ptéridophytes plus particulièrement la *cyathea aethiopica,* tel que défini précédemment en une quantité représentant de 0,0001% à 20 % du poids total de la composition et préférentiellement en une quantité représentant 0,001 % à 8 % du poids total de la composition.

Les compositions selon l'invention sont des compositions à usage pharmaceutique et peuvent se présenter sous toutes les formes galéniques normalement utilisées pour une administration orale. De façon connue, les compositions de l'invention peuvent contenir également des adjuvants habituels dans les domaines correspondants, tels que les gélifiants hydrophiles ou lipophiles, les conservateurs, les antioxydants, les matières colorantes. Par ailleurs, ces compositions peuvent contenir des actifs hydrophiles ou lipophiles. Les quantités de ces adjuvants ou actifs sont celles classiquement utilisées dans le domaine pharmaceutique et par exemple de 0,01 % à 20 % du poids total de la composition.

Quand les compositions de l'invention sont destinées à une application par voie orale, elles peuvent se présenter sous formes galéniques habituelles dans ce domaine, telles que les comprimés, les gélules, les produits buvables, notamment constitués extemporanément, les granulés, les poudres, dans les excipients habituels pour une telle application.

Un procédé de traitement pharmaceutique destiné à stimuler les défenses immunitaires, à lutter contre les infections bactériennes et virales consistant à ingérer une composition comprenant au moins un extrait d'au moins une graminée, plus un extrait d'au moins une rutacée, plus un extrait d'au moins d'une ptéridophyte telle que décrite précédemment est également décrit.

Les exemples et compositions suivants illustrent l'invention sans la limiter aucunement. Dans les compositions, les proportions indiquées sont en pourcentages en poids sauf mention contraire.

### Exemple 1 : Préparation d'un extrait paspalum conjugatum.

Deux cent grammes de la plante fraîche sont récoltés puis mélangé avec 80 ml d'eau non limitatif de préférence déminéralisée. L'ensemble est broyé et chauffé à 10°C environs pendant 20 à 30 minutes. Le broyat est ensuite pressé pour récolter le jus qui est ensuite filtré de préférence par une filtration stérilisante non limitatif. L'extrait ainsi obtenu est conservé à 4°C. Il renferme environ 15 g de matière sèche par litre et peut être lyophilisé pour obtenir un extrait sec. La filtration stérilisante évite la dégradation des actifs par les micro-organismes de l'environnement. Le véhicule d'eau facilite les formulations pharmaceutiques. L'extrait de *paspalum conjugatum* peut également être utilisé sous forme d'extrait sec.

### Exemple 2 : Préparation d'un extrait de fagara heitzii.

La préparation de *fagara heitzii* selon l'invention concerne particulièrement les écorces. Les écorces de *fagara heitzii* environ un kilogramme sont est mélangés avec 400 ml d'eau non limitatif de préférence de l'eau déminéralisée puis broyés au mixeur puis chauffés à plus de 100°C, ensuite refroidis avant d'extraire le jus par pression, puis filtré et stérilisé de préférence par une filtration stérilisante non limitatif. L'extrait ainsi obtenu est conservé à 4 °C. Il renferme environ 20 g de matière sèche par litre et peut être lyophilisé pour obtenir un extrait sec.

### Exemple 3 : Préparation d'un extrait de cyathea aethiopica.

La préparation de *cyathea aethiopica* selon l'invention concerne particulièrement toutes les parties aériennes de la plante. Environ 100 grammes de *cyathea aethiopica* sont mélangés avec 50 ml d'eau non limitatif de préférence de l'eau déminéralisée puis broyés au mixeur avant d'extraire le jus par pression, puis filtré et stérilisé de préférence par une filtration stérilisante non limitatif. L'extrait ainsi obtenu est conservé à 4 °C. Il renferme environ 15 g de matière sèche par litre et peut être lyophilisé pour obtenir un extrait sec.

Le dosage de la composition selon l'invention varie selon les symptômes et les traitements à administrer aux patients. Pour une administration orale pour les adultes, la dose usuelle de la composition est de 300 à 750 mg par jour, de préférence 500 mg par jour calculé comme les composants actifs, et repartis en deux prises de 250 mg le matin et le soir. Ce dosage permet d'observer les effets thérapeutiques de la composition.

La composition pharmaceutique antivirale, antibactérienne, antifongique et destinée à stimuler les défenses immunitaires selon l'invention comprend au moins un extrait d'au moins une graminée par exemple *paspalum conjugatum* et au moins un extrait d'au moins une rutacée par exemple la *fagara heitzii* et au moins un extrait d'au moins une ptéridophyte par exemple la *cyathea aethiopica* et au moins un excipient solide ou liquide. La composition destinée à l'administration orale est usuellement sous forme de poudre, de comprimés y compris les comprimés sublinguaux, l'émulsion, la préparation encapsulée, les granulés, les pilules, les préparations liquides y compris les extraits et les sirops. Les excipients solides ou liquides sont généralement connus pour les hommes du métier, de préférence chaque préparation contient une seule des excipients mentionnés dans l'exemple qui suit.

Les exemples des excipients utilisés pour les poudres et granulés ou autres préparations destinées à l'administration orale sont : le lactose, l'amidon, la dextrine, le phosphate de calcium, le carbonate de calcium, le silicate d'aluminium naturel ou synthétique, l'oxyde de magnésium, l'hydroxyde d'aluminium hydraté, le stéarate de magnésium, le bicarbonate de sodium.

Les préparations mentionnées plus ci-dessus peuvent être formulées par les méthodes conventionnelles. Les extraits de *paspalum conjugatum,* de *fagara heitzii,* de *cyathea aethiopica* selon l'invention pourront être utilisés comme des compléments alimentaires utiles pour garder une bonne santé sans pour autant produire l'effet thérapeutique. Dans ce cas, la préparation est souhaitable d'être administrée sous forme liquide, de granulés, de thé, des capsules ou autres formes usuelles.

### Exemple 4 : Exemples de formulations illustrant l'invention à titre indicatif et non limitatif. Ces formulations ont été obtenues par simple mélange des différents composants.

| Composition 1 : Préparation liquide des extraits des plantes | |
|---|---|
| 1- l'extrait de l'exemple 1 | 9 % |
| 2- l'extrait de l'exemple 2 | 65 % |
| 3- l'extrait de l'exemple 3 | 6 % |
| 4- le miel ou sucre | 20 % |
| | |

| Composition 2 : Gélules ou poudre | |
|---|---|
| 1- l'extrait de l'exemple 1 (extrait lyophilisé) | 15 % |
| 2- l'extrait de l'exemple 2 (extrait lyophilisé) | 77 % |
| 3- l'extrait de l'exemple 3 (extrait lyophilisé) | 8 % |
| | |

| Composition 3 : Gélules ou poudre | |
|---|---|
| 1- l'extrait de l'exemple 2 (extrait lyophilisé) | 64,36 % |
| 2- N-Acétylcystéine | 34,25% |
| 3- Sélénium | 0,03 % |
| 4- Zinc | 0,6 % |
| 5- Fer | 0,5 % |
| 6- Cuivre | 0,24 % |
| 7- Molybdène | 0,02 % |

### Evaluation Cliniques de la composition 1.

### Modalité de l'étude :

Elle a été mise en oeuvre dans les deux centres médicaux du docteur Pierre PYEBI-OYOUBI au Gabon, avec pour objectifs de tester l'efficacité de l'association des extraits de *paspalum conjugatum,* de la *fagara heitzii* et de la *cyathea aethiopica* sur les malades VIH-SIDA. Un test de dépistage de l'infection à VIH automatisé (VIDAS HIV DUO ou VT) a été réalisé avant et après le traitement à l'association des extraits de trois plantes. Le système VIDAS est basé sur la détection combinée de l'antigène P24 de HIV-1 et des immunoglobulines G anti-VIH1 et anti-VIH2 dans le sérum par la technique ELFA. Les maladies opportunistes au SIDA ont été également évaluées avant et après le traitement.

Un bilan médical est effectué avant et après un traitement de 1 à 6 mois. La numération-formule sanguine (NFS) a été également réalisée avant et après le traitement.

La NFS ou hémogramme permet de mesurer le nombre d'éléments de chacune des trois catégories de cellules sanguines que sont les globules rouges (hématies), les globules blancs (leucocytes) et les plaquettes. La numération globulaire consiste à compter le nombre de globules de chaque catégorie, et la formule leucocytaire à répartir les globules blancs en classes différentes en fonction de leurs caractéristiques. Ces différentes informations seront utiles, par exemple, pour diagnostiquer une anémie.

Ces examens apportent des informations fondamentales, et ils sont demandés systématiquement lors d'un bilan, en particulier lorsque l'on suspecte une anémie (baisse des globules rouges) ou une infection. Les résultats varient également en fonction de nombreux éléments dont la prise de médicaments.

Un groupe douze personnes séropositives ont été sélectionnées pour cette étude. Les résultats obtenus sont les suivants :

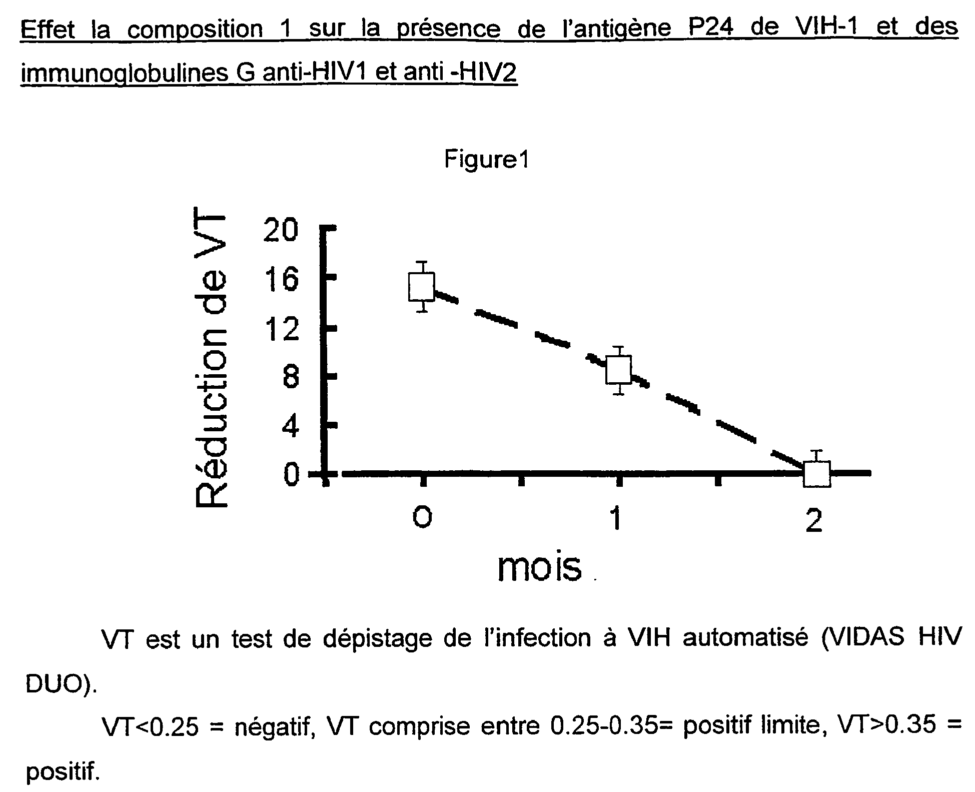

Les résultats obtenus montrent que l'administration par voie orale de la composition 1 aux malades VIH-Sida provoque une réduction significative de la présence dans le sérum des malades de l'antigène P24 de HIV-1 et des immunoglobulines G anti-VIH1 et anti-VIH2. Les résultats obtenus montrent en outre une rémission significative des effets cliniques des maladies opportunistes au VIH-Sida. Ces résultats suggèrent que le traitement des malades VIH-Sida par la composition 1, provoque une stimulation des défenses immunitaires, et une diminution des effets secondaires de l'infection aux virus VIH-Sida.

## Revendications

1. Composition pharmaceutique renfermant en tant que principe actif au moins un extrait d'au moins une graminée, mélangé avec au moins un extrait d'au moins une rutacée, mélangé avec au moins un extrait d'au moins une ptéridophyte, lesdits extraits étant des extraits de *paspalum conjugatum,* de *fagara heitzii* et de *cyathea aethiopica.*

2. Composition selon la revendication 1, **caractérisée en ce qu'**elle est destinée à stimuler les défenses immunitaires.

3. Composition selon la revendication 2, **caractérisée en ce qu'**elle est destinée à stimuler les défenses immunitaires affaiblies par la maladie du VIH-SIDA.

4. Composition selon la revendication 1, **caractérisée en ce qu'**elle est destinée à être utilisée en tant que médicament antibactérien ou antiviral.

5. Composition selon la revendication 1, **caractérisée en ce qu'**elle est destinée à être utilisée comme antifongique.

6. Composition selon la revendication 1, **caractérisée en ce que** lesdits extraits de *paspalum conjugatum* sont utilisés sous forme sèche ou lyophilisée en une quantité représentant 0,0001 % à 30% du poids total de la composition, et que lesdits extraits de *fagara heitzii* sont utilisés sous forme sèche ou lyophilisée en une quantité représentant 0,0001% à 99% du poids total de la composition, et que lesdits extraits de *cyathea aethiopica* sont utilisés sous forme sèche ou lyophilisée en une quantité représentant 0,0001% à 20% du poids total de la composition.

7. Composition selon la revendication 1, **caractérisée en ce que** dans la composition, lesdits extraits de *paspalum conjugatum* sont utilisés sous forme liquide en une quantité représentant 0,0001% à 30% du poids total de la composition et que lesdits extraits de *fagara heitzii* sont utilisés sous forme liquide en une quantité représentant 0,0001% à 99% du poids total de la composition, et que lesdits extraits de *cyathea aethiopica* sont utilisés sous forme liquide en une quantité représentant 0,0001 % à 20% du poids total de la composition.

8. Composition selon l'une des revendications 1 à 7, **caractérisée en ce qu'**elle est utilisée par voie orale pour stimuler les défenses immunitaires affaiblies par les maladies virales, VIH-SIDA, pour lutter contre les infections virales et contre les infections bactériennes.

## Claims

1. Pharmaceutical composition containing as active ingredient at least one extract of at least one gramineous plant, mixed with at least one extract of at least one rutaceous herb, mixed with at least one extract of at least one pteridophyte, said extracts being extracts of *paspalum conjugatum, fagara heitzii* and *cyathea aethiopica.*

2. Composition according to claim 1, **characterized in that** it is intended to stimulate the immune defences.

3. Composition according to claim 2, **characterized in that** it is intended to stimulate immune defences weakened by the disease HIV-AIDS.

4. Composition according to claim 1, **characterized in that** it is intended to be used as an antibacterial or antiviral medicament.

5. Composition according to claim 1, **characterized in that** it is intended to be used as an antifungal.

6. Composition according to claim 1, **characterized in that** said extracts of *paspalum conjugatum* are used in dry or lyophilized form in a quantity representing 0.0001% to 30% of the total weight of the composition and that said extracts of *fagara heitzii* are used in dry or lyophilized form in a quantity representing 0.0001% to 99% of the total weight of the composition, and that said extracts of *cyathea aethiopica* are used in dry or lyophilized form in a quantity representing 0.0001% to 20% of the total weight of the composition.

7. Composition according to claim 1, **characterized in that** in the composition said extracts of *paspalum conjugatum* are used in liquid form in a quantity representing 0.0001% to 30% of the total weight of the composition and that said extracts of *fagara heitzii* are used in liquid form in a quantity representing 0.0001% to 99% of the total weight of the composition, and that said extracts of *cyathea aethiopica* are used in liquid form in a quantity representing 0.0001% to 20% of the total weight of the composition.

8. Composition according to one of claims 1 to 7, **characterized in that** it is used by oral route to stimulate immune defences weakened by the viral diseases HIV-AIDS, to fight viral infections and bacterial infections.

## Patentansprüche

1. Pharmazeutische Zusammensetzung, die als Wirkstoff mindestens einen Extrakt mindestens einer Graminee enthält, der mit mindestens einem Extrakt mindestens einer Rutacee gemischt ist, der mit mindestens einem Extrakt mindestens eines Pteridophyten gemischt ist, wobei diese Extrakte Extrakte von *Paspalum conjugatum, Fagara heitzii* und *Cyathea aethiopica* sind.

2. Zusammensetzung nach Anspruch 1, **dadurch gekennzeichnet, dass** sie zur Stimulierung der Immunabwehr bestimmt ist.

3. Zusammensetzung nach Anspruch 2, **dadurch gekennzeichnet, dass** sie zur Stimulierung der durch die Krankheit HIV/AIDS geschwächten Immunabwehr bestimmt ist.

4. Zusammensetzung nach Anspruch 1, **dadurch gekennzeichnet, dass** sie zur Verwendung als antibakterielles oder antivirales Medikament bestimmt ist.

5. Zusammensetzung nach Anspruch 1, **dadurch gekennzeichnet, dass** sie zur Verwendung als Antimykotikum bestimmt ist.

6. Zusammensetzung nach Anspruch 1, **dadurch gekennzeichnet, dass** die Extrakte von *Paspalum conjugatum* in trockener oder lyophilisierter Form in einer Menge verwendet werden, die 0,0001 % bis 30 % des Gesamtgewichts der Zusammensetzung darstellt, und dass die Extrakte von *Fagara* heitzii in trockener oder lyophilieserter Form in einer Menge verwendet werden, die 0,0001 % bis 99 % des Gesamtgewichts der Zusammensetzung darstellt, und dass die Extrakte von *Cyathea aethiopica* in trockener oder lyophilisierter Form in einer Menge verwendet werden, die 0,0001 % bis 20 % des Gesamtgewichts der Zusammensetzung darstellt.

7. Zusammensetzung nach Anspruch 1, **dadurch gekennzeichnet, dass** in der Zusammensetzung die Extrakte von *Paspalum conjugatum* in flüssiger Form in einer Menge verwendet werden, die 0,0001 % bis 30 % des Gesamtgewichts der Zusammensetzung darstellt, und dass die Extrakte von *Fagara* heitzii in flüssiger Form in einer Menge verwendet werden, die 0,0001 % bis 99 % des Gesamtgewichts der Zusammensetzung darstellt, und dass die Extrakte von *Cyathea aethiopica* in flüssiger Form in einer Menge verwendet werden, die 0,0001 % bis 20 % des Gesamtgewichts der Zusammensetzung darstellt.

8. Zusammensetzung nach einem der Ansprüche 1 bis 7, **dadurch gekennzeichnet, dass** sie auf oralem Weg verwendet wird, um die durch die Viruserkrankungen, HIV/AIDS, geschwächte Immunabwehr zu stimulieren, um virale Infektionen und bakterielle Infektionen zu bekämpfen.
